# EUROPEAN PATENT APPLICATION

(11) **EP 4 082 495 A1**
(43) Date of publication of application: **02.11.2022**
(21) Application number: 21171039.7
(22) Date of filing: 28.04.2021
(51) Int. Cl.: A61F 13/15, A61F 13/514, B32B 7/14, B32B 25/10, B32B 27/12

(54) **LAMINATE FOR AN ABSORBENT ARTICLE, ABSORBENT ARTICLE COMPRISING SAID LAMINATE AND PROCESS FOR MAKING SUCH LAMINATE**

(71) Applicant: Ontex BV, 9255 Buggenhout (BE); Ontex Group NV, 9320 Erembodegem (BE)
(72) Inventor: Heege, Thomas, 56761 Düngenheim (DE); Iffland, Dirk, D-45529 Hattingen (DE); Lambertz, Christina, 56566 Neuwied (DE); Granado, Martín, 56727 Mayen (DE); WEBER, Ainas, 53474 Bad Neuenahr-Ahrweiler (DE)
(74) Representative: Saurat, Thibault

(57) **Abstract**

The current invention concerns a laminate (2) for an absorbent article, preferably to be used as the backsheet of said absorbent article, said laminate (2) comprising:
- a first layer (4) comprising a fibrous material such as a nonwoven material;
- a second layer (6) comprising a film;
- a third layer (8) comprising an adhesive;
- the third layer (8) being arranged between the first (4) and second (6) layer;
wherein the adhesive in the third layer (8) is applied in a pattern, said pattern comprising:
- a first sub-pattern (10) comprising a frame (12) following the perimeter of the laminate (2) and defining an inner surface, said first sub-pattern (10) comprising a first amount of adhesive;
- a second sub-pattern (14) arranged within the inner surface of the frame (12), said second sub-pattern (14) comprising a second amount of adhesive, the second amount of adhesive being less than the first amount of adhesive.

The invention also concerns an absorbent article comprising such laminate and a process for manufacturing such laminate.

## Description

### TECHNICAL FIELD

The invention pertains to the technical field of absorbent hygiene products. In particular, the present invention relates to a laminate for an absorbent article for absorbing body fluids, said laminate preferably being used as the backsheet of said absorbent article. The invention also pertains to an absorbent article comprising such laminate as well as the process for making such laminate.

The absorbent articles may in particular be baby and toddler diapers (including training pants), feminine sanitary pads and/or adult incontinence articles. The present invention relates more particularly to the nature of the backsheet of these articles.

### BACKGROUND

Absorbent articles such as baby and toddler diapers (including training pants), feminine sanitary pads and/or adult incontinence articles, generally comprise several different layers that are bonded together. For example, an absorbent article can comprise a topsheet, facing inward and corresponding to the body-facing layer, a backsheet facing outward and corresponding to the garment-facing layer and an absorbent core arranged in between said topsheet and backsheet. These layers can be bonded through different technics such as embossment, lamination, welding, extrusion but a layer of adhesive will be present most of the time to ensure the preservation of the structural construct.

More specifically, disposable diapers conventionally include a chassis having a liquid permeable topsheet, a liquid impermeable which can also be breathable backsheet and an absorbent structure sandwiched between the topsheet and backsheet. The chassis has a front body panel which, in use, extends over the stomach and front hip area of the user, and a rear body panel which, in use, extends over the back and the rear hip area of the user. Each of the body panels has a waist portion such that, when the diaper is fastened around the waist of the user, the waist portions provide a continuous encirclement of the user. In order to fasten the diaper around the waist of a user, a fastening system comprising fastening tabs is commonly employed. Fastening tabs may be provided on side panels which extend from lateral side edges of the diaper chassis.

Disposable pants have a similar construction but typically comprise front and back elasticized belts at either end of the absorbent structure and are sealed together at lateral side seams to form an underwear-resembling product that can be worn by a subject by pulling it up over the legs and may be removed either by pulling it down in the opposite direction or by tearing the side seams.

Usually, the layer of adhesive is continuously sprayed or coated on a continuous moving web corresponding to one of the layers of the absorbent articles. The quality of the bond between the components of the absorbent article is generally dependent upon the amount of adhesive and the type of the adhesive.

More specifically, backsheet materials for absorbent articles such as diapers are normally formed by a laminate of a very thin plastic film with a nonwoven web where the nonwoven web is oriented outside of the article (on the garment facing side) so as to block the fluid while having a soft touch feel. The two materials are called backsheet film and backsheet nonwoven web respectively. These two materials are bonded, or laminated, by a layer of adhesive in-between.

On one hand, by applying a continuous layer of adhesive on the substrate, usually the thin plastic film, the resulting final product will present a rugged surface and will not feel comfortable to the person touching the product. On the other hand, an article where the layer of adhesive is in insufficient amount will affect the quality of the bonding of the backsheet and can cause failing, delamination and tearing of the backsheet. Therefore, a balance must be found between the film properties, the amount of glue applied in the backsheet laminate between backsheet film and backsheet nonwoven web, each material tensile properties and the glue bonding strength to help withstand the stresses in highspeed manufacturing.

There remains a need for an improved backsheet that presents a proper balance between the soft touch feel while maintaining its structural integrity.

### SUMMARY OF THE INVENTION

The present invention concerns a laminate for an absorbent article, preferably consisting of a backsheet of said absorbent article, said laminate comprising:
- a first layer comprising a fibrous material such as a nonwoven fabric, web or material;
- a second layer comprising a film such as a polymer film;
- a third layer comprising an adhesive;
- the third layer being arranged between the first and second layer;
wherein the adhesive in the third layer is applied in a pattern, said pattern comprising:
- a first sub-pattern comprising a frame following the perimeter of the laminate and defining an inner surface, said first sub-pattern comprising a first amount of adhesive;
- a second sub-pattern arranged within the inner surface of the frame, said second sub-pattern comprising a second amount of adhesive, the second amount of adhesive being less than the first amount of adhesive.

The term "laminate" used herein refers to an element that was manufactured by uniting superposed layers of one or more material, these layers being united by an adhesive.

The term "film" used herein refers to a thin layer of material, such as polymer, bioplastic, thermoplastic starch, biopolymer such as PolyLactic Acid (PLA), PolyHydroxyButyrate (PHB), plastic, bio-based polyethylene, or any impermeable material adapted to be used in an absorbent article, a thin film, or thin layer, implies a thickness less than 0.2 mm, for example a thickness of less than 0.1 mm.

The first sub-pattern enables to have a good bonding strength and ensures the structural integrity of the absorbent article whereas the second sub-pattern maintains a certain bonding strength while ensuring a soft touch feel.

According to an embodiment, the first amount of adhesive is at least five times greater than the second amount of adhesive, for example ten times greater than the second amount of adhesive.

According to an embodiment, the first amount of adhesive is comprised between 0,5 and 5 gsm.

According to an embodiment, the second amount of adhesive is under 0.1 gsm.

According to an embodiment, the first sub-pattern comprising a frame comprises at least one additional reinforcement zone, comprising a third amount of adhesive that is greater than the first amount of adhesive.

According to an embodiment, the third amount of adhesive is at least two times greater than the first amount of adhesive.

According to an embodiment, the second sub-pattern comprises discrete areas of adhesive, the discrete areas of adhesive being arranged in a staggered configuration or in an even distribution.

According to an embodiment, the second sub-pattern comprises discrete areas of adhesive, the discrete areas of adhesive being in shape of dots or rectangles.

According to an embodiment, the first sub-pattern occupies between 10 and 40 % of the total surface of the laminate whereas the inner area occupies between 60 and 90 % of the total surface of the laminate.

According to an embodiment, the pattern of the third layer comprises a third sub-pattern arranged at least partially in-between the first and second sub-pattern, said third sub-pattern comprising a fourth amount of adhesive that is greater than the second amount of adhesive and less than the first amount of adhesive.

According to an embodiment, the laminate extends longitudinally in a MD direction and laterally in a CD direction, said laminate comprising a front end and a rear end arranged at each longitudinal end and two lateral sides arranged at each lateral end, and
wherein the frame is divided in at least four panels having polygonal shape: a front pan, a rear panel and two side panels,
- wherein the front and rear panels extend more in the lateral direction than in the longitudinal direction and
- the side panels extend more in the longitudinal direction than in the lateral direction.

According to an embodiment, the at least one reinforcement zone is arranged in the rear panel. In other terms, the at least one reinforcement zone is arranged on the rear panel. In other terms, the at least one reinforcement zone is arranged on, or in, or at, the rear panel. According to an embodiment, the panels are joined to one another with a bevel arranged on, or at, the inner contour of the frame. In other terms, the panels are joined to one another with a bevel arranged on, or at, the inner contour of the frame.

The invention also pertains to an absorbent article comprising a topsheet, a backsheet comprising a laminate as described above and an absorbent core arranged in between said topsheet and said backsheet.

The invention also pertains to a process for manufacturing a laminate as described above comprising the step of:
- providing respectively the second layer of film or the first layer of nonwoven fabric;
- applying the third layer of adhesive respectively onto said second layer or onto said first layer according to the first and second sub-pattern at least preferably with, or by, an electromagnetic actuator;
- depositing respectively said first layer or second layer onto the third layer of adhesive.

In other words, the process for manufacturing a laminate as described above comprises the step of:
- providing the second layer comprising a film;
- applying the third layer of adhesive onto said second layer according to the first and second sub-pattern at least preferably with, or by, an electromagnetic actuator;
- depositing the first layer comprising a nonwoven fabric onto the third layer of adhesive,
   or,
- providing the first layer comprising a nonwoven fabric;
- applying the third layer of adhesive onto said first layer according to the first and second sub-pattern at least preferably with, or by, an electromagnetic actuator;
- depositing said second layer comprising a film onto the third layer of adhesive.

According to an embodiment, the process comprises an additional step of applying fastening tabs onto the third layer in an overlapping zone in said reinforcement zone.

All these embodiments mentioned above can be taken individually or in combination.

Further embodiments are described below and in the claims.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**FIG. 1** illustrates a cross-sectional view schematic of the laminate according to the invention.
**FIG. 2** illustrates a top view schematic of a part of the laminate according to an embodiment of the disclosure.
**FIG. 3** illustrates a top view schematic of a part of the laminate according to another embodiment of the disclosure.
**FIG. 4** illustrates a top view schematic of a part of the laminate according to another embodiment of the disclosure.
**FIG. 5** illustrates a top view schematic of a part of the laminate according to another embodiment of the disclosure.
**FIG. 6** illustrates a top view schematic of a part of the laminate according to another embodiment of the disclosure.
**Fig. 7** illustrates schematically from a side view the apparatus and process to manufacture the laminate.
**FIG. 8** illustrates an isometric schematic view of an absorbent article.
**FIG. 9** illustrates a top view schematic of a part of the laminate according to another embodiment of the disclosure.
**FIG. 10** illustrates a top view schematic of a part of two laminates units according to another embodiment of the disclosure.

### DETAILED DESCRIPTION OF THE INVENTION

The present invention concerns a laminate for an absorbent article, preferably a laminate used as the backsheet of said absorbent article. The invention also concerns an absorbent article comprising said laminate as well as the process for making such laminate.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints except where otherwise explicitly stated by disclaimer and the like.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

Preferably, a diaper comprises a liquid permeable "topsheet", a liquid impermeable "backsheet", and an "absorbent medium or core" disposed between the top sheet and the backsheet. The topsheet, backsheet and the absorbent medium could be made from any suitable material known to the person skilled in the art. The topsheet is generally located at or near the bodyside surface of the article, i.e. inward, while the backsheet is generally located at or near the garment-side surface of the article, i.e. outward. Optionally, the article may comprise one or more separate layers which are in addition to the backsheet and are interposed between the backsheet and the absorbent medium. Topsheet and backsheet are connected or otherwise associated together in an operable manner.

The "absorbent medium" or "absorbent core" or "absorbent body" is the absorbent structure disposed between the topsheet and the backsheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hotmelt, water or solvent borne adhesive that can be applied to a surface of a film layer in the required pattern or network of adhesive areas to form the film-nonwoven laminate of the present invention. Accordingly, suitable adhesives include conventional hotmelt adhesives, pressure-sensitive adhesives, rubber-based adhesives such as Ethlylene Propylene Rubber (EPM) or EDPM, stretch adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications using a pneumatic, piezo or electromagnetic actuator. Further, such adhesive may be applied within a product component and then exposed to pressure such that contact of a second product component with the adhesive containing product component forms an adhesive bond between the two components.

The term "rear end" is used in reference to a longitudinal direction and refers to the area of the absorbent article which is contact with the back of the wearer when the absorbent article is worn.

The term "backsheet" refers to a material forming a liquid impermeable cover of the absorbent article. The backsheet prevents the exudates contained in the absorbent structure from wetting articles such as bedsheets and overgarments which contact the disposable absorbent article. The backsheet comprises a composite layer composed of multiple components assembled side-by-side or laminated. The backsheet may be the same or different in different parts of the absorbent article. At least in the area of the absorbent medium the backsheet comprises a liquid impervious material in the form of a thin plastic film, e.g. a polyethylene or polypropylene film, a nonwoven material which may be coated with a liquid impervious material, a hydrophobic nonwoven material, which resists liquid penetration, or a laminate of a plastic film and a nonwoven material. The backsheet material may be breathable so as to allow vapour to escape from the absorbent material, while still preventing liquids from passing there through. Examples of breathable backsheet materials are porous polymeric films, nonwoven laminates of spunbond and meltblown layers and laminates of porous polymeric films and nonwoven materials.

The present invention pertains to a laminated that is preferably used as a backsheet for an absorbent article, the terms "laminate" and "backsheet" are thereby equivalent.

The term "front end" is used in reference to a longitudinal direction and refers to the area of the absorbent article which is contact with the belly of the wearer when the absorbent article is worn.

As used herein, the "body-facing", "inward" or "bodyside" surface means that surface of the article or component which is intended to be disposed toward or placed adjacent to the body of the wearer during ordinary use, while the "outward", "outward-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to layers, preferably films or elastic laminates, having a water vapor transmission rate (WVTR) of at least 300 grams/m² - 24 hours.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

Further, an absorbent article can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polymer, biopolymers, bioplastic, polypropylene, polyester, rayon, nylon, foams, plastic films, formed films, and elastic foams. A number of manufacturing techniques may be used to manufacture the containment flaps. For example, the containment flaps may be woven, nonwoven, spunbonded, carded, cast, blown or the like.

An absorbent article can comprise leg containment gaskets. Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the body-facing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.

"Mechanical bond" is an attachment between two or more elements, components, regions, or webs and may comprise heat bonds, pressure bonds, ultrasonic bonds, dynamic mechanical bonds, or any other suitable non-adhesive attachment means or combinations of these attachment means.

"Hotmelt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. For example, a typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The terms "Discrete bonding pattern" or "Discontinuous bonding pattern" as used herein as synonyms and refers to a pattern of bonding areas, in particular bonding areas between layers, whereby at least in at least one region the layers are not bonded. A discontinuous bonding pattern may comprise one or more connected bonding areas or multiple disconnected bonding areas. A discontinuous bonding pattern further may comprise a connected bonding area comprising a number of holes, where the layers are not bonded, according to a regular pattern, or it may comprise discrete disconnected bonding areas, e.g. a point bonded pattern which comprises a plurality of separate bonding points surrounded by unbonded areas or a line-bonded pattern which comprises a plurality of separate bonding lines alternated by unbonded areas, according to a regular pattern.

The term "discrete areas of adhesive" is equivalent to the term "discrete bonding areas" and refers to the application, or deposit, of adhesive on a given area in order to bond or join two layers.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" or "elastified" refers to a material, layer, or substrate that is naturally non-elastic, but which has been rendered elastic by, for example, suitably joining an elastic material, layer, or substrate thereto.

"Elongation" means the ratio of the extension of a material to the length of the material prior to the extension (expressed in percent). "Extension" means the change in length of a material due to stretching (expressed in units of length).

The term "finished" or "final", when used with reference to a product, means that the product has been suitably manufactured for its intended purpose.

As used herein, the term "garment" means any type of apparel which may be worn. This includes diapers, training pants, incontinence products, surgical gowns, industrial workwear and coveralls, undergarments, pants, shirts, jackets and the like.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The use of the term "layer" can refer, but is not limited, to any type of substrate, such as a woven web, nonwoven web, films, laminates, composites, elastomeric materials, or the like, or even formulations, such as adhesives, that form a substrate upon a change in conditions (e.g. solidification of a hotmelt adhesive when the temperature drops below a predetermined amount). A layer can be liquid and air permeable, permeable to air but impermeable to liquids, impermeable both to air and liquid, or the like. When used in the singular, it can have the dual meaning of a single element or a plurality of elements.

The term "nonwoven fabric", "nonwoven material", "nonwoven layer" or "nonwoven web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

Use of the term "substrate" includes, but is not limited to, woven or nonwoven webs, porous films, ink permeable films, paper, composite structures, or the like.

Superabsorbent materials (e.g. superabsorbent polymers) suitable for use in the present disclosure are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to about 60% by weight.

Typically, the superabsorbent material, when present, will be included in an amount of about 5% to about 40% by weight, based on the total weight of the absorbent layer.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

The term "topsheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured polymer films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction.

"Substantial portion" as used herein with reference to an element/component (adhesive pattern), refers to at least 80%, preferably at least 90%, more preferably at least 95%, of said element/component, generally measured as a surface area taken in the laid flat state of the absorbent article.

Embodiments according to the disclosure will now be described. It is understood that technical features described in one or more embodiments maybe combined with one or more other embodiments without departing from the intention of the disclosure and without generalization therefrom.

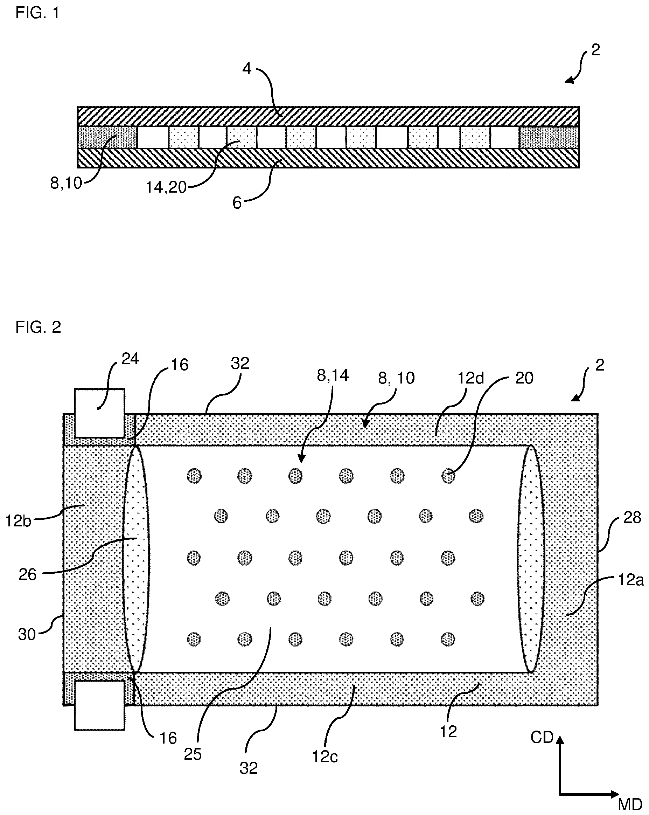

FIG. 1 illustrates a cross-sectional view of a laminate 2 for an absorbent article according to the invention, said laminate 2 is preferably used as the backsheet of an absorbent article.

The laminate 2 comprises a first layer 4 comprising a fibrous material such as nonwoven material, or nonwoven fabric. The first layer 4 can comprise other materials including and not limited to pigments, antioxidants, stabilizers, surfactants, waxes, flow promoters, solvents, particulates or materials added to enhance processability of the composition. The laminate 2 comprises a second layer 6 comprising a film such as a thin film of polymeric material comprising material such as PLA, PHB, thermoplastic starch, bio-sourced polyethylene (bio-PE) and/or polypropylene (PP). As illustrated in FIG. 1, the second layer 6 is arranged under, or on the bottom of, the first layer 4. The laminate 2 further comprises a third layer 8 comprising an adhesive such as a hotmelt adhesive, the third layer 8 being arranged between the first 4 and second 6 layer. As illustrated in FIG. 1, the laminate 2 corresponds to a superposition of a first 4, second 6 and third 8 layers, wherein the first 4 and second 6 layers are united, or bonded, by the third layer 8 of adhesive. In other words, according to the invention, the first 4 and second 6 layer are joined by adhesive bonding.

According to the invention, the adhesive in the third layer 8 is applied in a pattern as exemplified in FIG. 2. FIG. 2 illustrates a top view schematic of a part of the laminate 2 in which the first layer 4 was removed for illustrative purposes. The adhesive pattern of the third layer 8 comprises a first sub-pattern 10, said first sub-pattern comprising a frame 12 following the perimeter of the laminate 2, for example following the entirety of the periphery of the laminate 2, and defining an inner surface, or a void in its central region, said first sub-pattern 10 comprising a first amount of adhesive. The adhesive pattern of the third layer 8 comprises a second sub-pattern 14 arranged within the inner surface of said frame 12, said second sub-pattern 14 comprising a second amount of adhesive. The first amount of adhesive is greater than the second amount of adhesive. In other words, the first sub-pattern 10 surrounds the second sub-pattern.

As explained above and as exemplified in FIG. 2 to FIG. 6, the first sub-pattern 10 is in the shape of a frame 12 having at least four panels 12a,12b,12c,12d joined together and thus defining an inner surface, or inner void, said first sub-pattern 10 following the periphery, in other words the outer contour, of the laminate 2 or backsheet. In other words, the laminate 2 corresponds to a sheet with borders, or an outline, that extends in a longitudinal (MD) and a lateral (CD) direction, the first sub-pattern 10 of adhesive is applied on the rim of said sheet and follows the periphery of this rim, or edge.

This way, the first amount of adhesive ensures the structural integrity of the backsheet 2, while the second amount of adhesive enables a soft touch feel of the backsheet 2. By reducing the amount of adhesive in the central part of the laminate 2, it is also possible to save on raw material. The laminate 2 and particularly the first layer 4 can then be arranged on the outer side or garment-facing side, for example if the absorbent article corresponds to a disposable diaper, the caretaker will feel a softer material when touching the article, specifically the backsheet 2, before disposing of it.

The amount of adhesive is expressed herein in grams (of adhesive) per square meter (of laminate or backsheet) (g/m²) and will be referenced as gsm from hereon.

According to an embodiment, the first amount of adhesive, meaning the amount of adhesive of the first sub-pattern 10, is at least five times greater than the second amount of adhesive, meaning the amount of adhesive of the second sub-pattern. The first amount of adhesive can be 5, 6, 7, 8, 9, 10, 11, 12, 13, 14, 15, 20, any decimal value in-between 10 and 20, or more times greater than the second amount of adhesive.

According to an embodiment, the first amount of adhesive is comprised between about 1 and about 10 gsm. In other words, for each square meter of laminate 2 or backsheet 2 there is between about 1 gram and about 10 grams of adhesive in the first sub-pattern 10. The first amount of adhesive can comprise about 0,5; 0,6; 0,7; 0,8; 0,9; 1; 1,1; 1,2; 1,3; 1;4; 1.5; 1,6; 1,7; 1,8; 1,9; 2; 2,1; 2,2; 2,3; 2;4; 2.5; 2,6; 2,7; 2,8; 2,9; 3; 3,1; 3,2; 3,3; 3;4; 3.5; 3,6; 3,7; 3,8; 3,9; 4; 5; 6; 7; 8; 9; 10 gsm of adhesive or any amount in between 0,5 and 10 gsm.

According to an embodiment, the second amount of adhesive is about 0.1 gsm or lower than 0.1 gsm, meaning that for each square meter of backsheet there is about 0.1 gram or less of adhesive in the second sub-pattern 14.

According to an embodiment, the first sub-pattern 10, or in other terms the frame 12, comprises at least one reinforcement zone 16, comprising a third amount of adhesive that is greater than the first amount of adhesive. As illustrated in FIG. 2, the first sub-pattern comprises two reinforcement zones 16 each comprising a third amount of adhesive.

According to an embodiment, the third amount of adhesive, meaning the amount of adhesive present in the reinforcement zone(s) 16, is at least two times greater than the first amount of adhesive. The third amount of adhesive can be 2; 2,1; 2,2; 2,3; 2;4; 2.5; 2,6; 2,7; 2,8; 2,9; 3; 3,1; 3,2; 3,3; 3;4; 3.5; 3,6; 3,7; 3,8; 3,9; 4; 5; 6; 7; 8; 9; 10 or more times greater than the first amount of adhesive. The third amount of adhesive can comprise 1; 1,1; 1,2; 1,3; 1;4; 1.5; 1,6; 1,7; 1,8; 1,9; 2; 2,1; 2,2; 2,3; 2;4; 2.5; 2,6; 2,7; 2,8; 2,9; 3; 3,1; 3,2; 3,3; 3;4; 3.5; 3,6; 3,7; 3,8; 3,9; 4; 5; 6; 7; 8; 9; 10; 11; 12; 13; 14; 15; 16; 17; 18; 19; 20 gsm of adhesive. The reinforcement zone(s) 16 is configured to enable the fixation of fastening tabs 24 or ears onto the backsheet 2 as it will be explained afterward.

As explained above, the second sub-pattern 14 comprises a second amount of adhesive that is less than the first amount of adhesive, as well as the third amount of adhesive. To achieve this, the second sub-pattern 14 can for example be applied in a discontinuous or discrete pattern. The second sub-pattern 14 comprises a discrete, or intermittent, bonding pattern. In other words, the adhesive is deposited onto the first 4 or second layer 6 so as to form discrete areas 20 of adhesive, or discontinuous areas 20 of adhesive, on the nonwoven fabric or on the film.

The adhesive of the third layer 8 is applied in a first sub-pattern 10 in the shape of a frame 12. The adhesive of the third layer 8 is also applied in a second sub-pattern 14, in the shape of a discontinuous bonding pattern, arranged within the first sub-pattern 10 and specifically within the inner surface defined by the frame 12, so that the bonding areas between the first 4 and second 6 layers, whereby in at least one region are not bonded. In other words, the second sub-pattern 14 comprises discrete bonding areas 20 and continuous or discontinuous unbonded areas 25.

In other words, the first bonding sub-pattern 10 is a continuous bonding pattern and the second sub-pattern is a discrete bonding pattern.

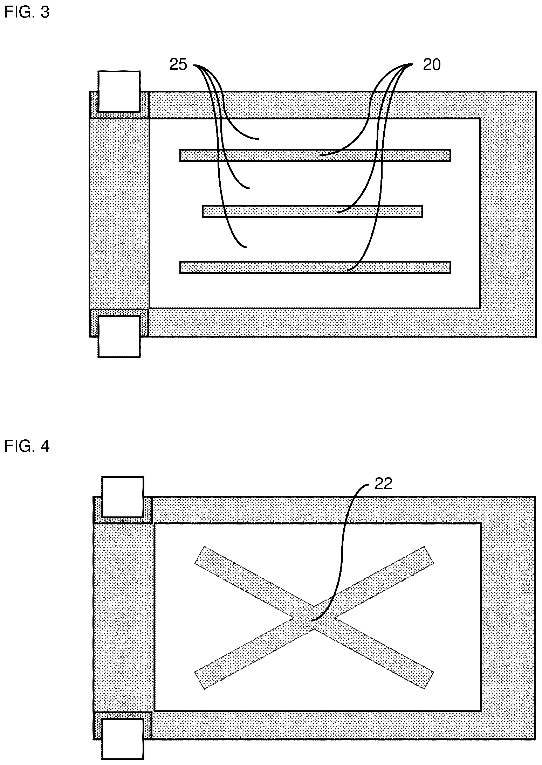

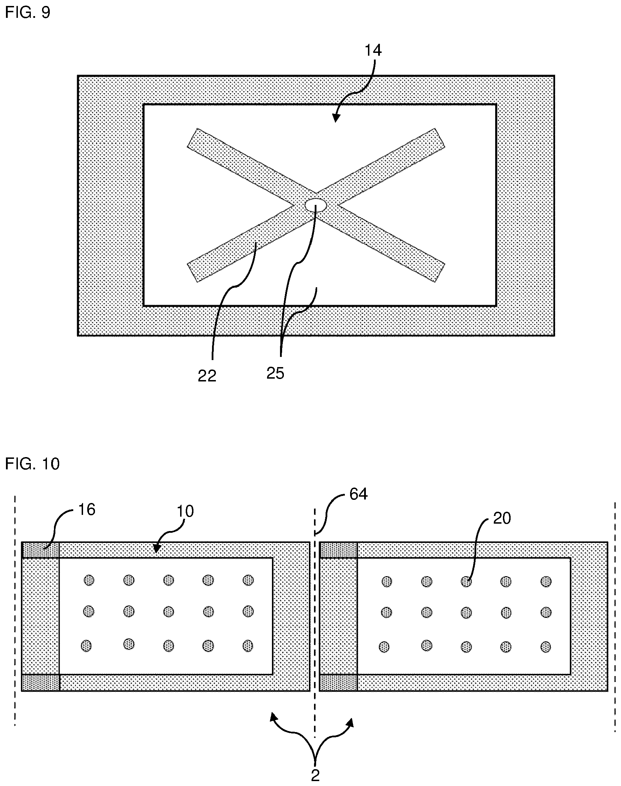

The discontinuous bonding pattern, i.e. second sub-pattern 14, may comprise one or more connected bonding area 22 (as illustrated in FIG. 4) and/or multiple discrete or disconnected bonding areas 20 (as exemplified in FIG. 2, FIG. 3, FIG. 5, FIG. 6). The second sub-pattern 14 may comprise a connected bonding area 22 comprising a number of holes (FIG. 9), where the layers 4, 6 are not bonded, according to a regular or irregular pattern, or it may comprise multiple discrete bonding areas 20, e.g. a dot-bonding pattern which comprises a plurality of separated bonding points 20 surrounded by unbonded areas 25 (FIG. 2) or a line-bonding pattern which comprises a plurality of separate bonding lines alternated by unbonded areas 25, according to a regular or irregular pattern (FIG. 3).

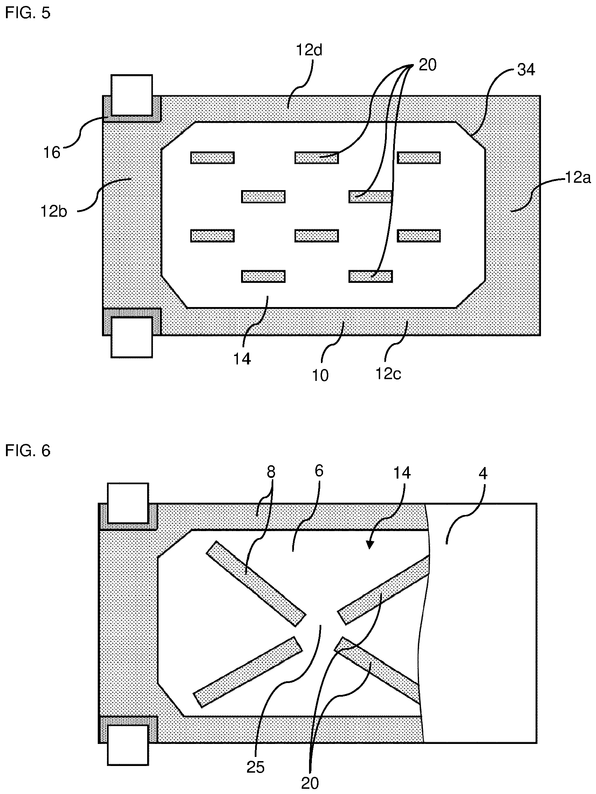

As illustrated in FIG. 2, the second amount of adhesive is applied here in dots that are separate from one another in a staggered arrangement. The discrete areas 20 of adhesive can be applied in dots, circles, ellipses, triangles, rectangles (as illustrated in FIG. 5), pentagon, hexagon, or any other polygonal shapes, the invention not being limited to the shape of these discrete areas. The discrete areas 20 can be arranged in a staggered configuration such as a checkerboard pattern, in even or uneven rows. In other words, the discrete areas 20 can be arranged in a staggered configuration, where the rows are aligned two by two with two adjacent rows being shifted by a gap or pitch. The rows can also be arranged in an irregular staggered configuration, where the shifting gap is different from one row to another. Alternatively, the discrete areas 20 can be applied in rows and columns that are evenly distributed as exemplified in FIG. 10.

It can also be said that the second sub-pattern 14 has a lower bond density than the first sub-pattern 10. The bond density can be distributed equally throughout the second sub-pattern 14, or alternatively, it can be distributed with a graded density. A equal bond density ensures a stronger bonding of the layer whereas the graded density can ensure a haptical benefit for specific regions of the absorbent article.

According to another embodiment as illustrated in FIG. 3, the second amount of adhesive is applied here in parallel elongated lines that are separated from one another. The discrete areas 20 of adhesive are in the shape of elongated lines or rows, the rows of adhesive being parallel to one another. In other words, the second sub-pattern 14 is in the shape of discrete elongated rows of adhesive. The invention is not limited to the shape of these discrete areas, for instance the lines of adhesive can be waved shape arranged in a congruent or non-congruent pattern, the lines can be curved, or arcuate, in a concave or convex shape. The lines can be continuous or dashed (as illustrated in FIG. 5). The dimensions (length, width) of these adhesive lines can be equal from one to another or the lines can have different dimensions, as illustrated in FIG. 3 where the discrete areas 20 are applied in three parallel rows, the row in the middle being slightly shorter than the two outer lines.

As explained above, the second sub-pattern 14 comprises a second amount of adhesive that is less than the first amount of adhesive as well as the third amount of adhesive. To achieve this, the second sub-pattern 14 can also be applied in a continuous pattern, meaning that the application of adhesive in the second sub-pattern 14 has at least one application of adhesive is linked to another application of adhesive and thus forming a continuity of adhesive. In other words, the adhesive is deposited onto the first 4 or second layer 6 so as to form one or more continuous areas 22 of adhesive, or connected areas of adhesive, on the nonwoven fabric or film. FIG. 4 illustrates such an example where the adhesive is applied in a cross-shape with two branches, or lines, of adhesive joined at their central portion forming a continuous area 22 of adhesive. Of course the invention is not limited to the shape of this connected area, for example, the adhesive can be applied in the form of, meaning in the shape of, an H, i.e. two parallel lines with a third perpendicular line of adhesive, or be applied with secant lines such as in the shape of a T, a X or a V, or in the shape of a hollow diamond, with four segments each segment intersecting with two other segments at their extremities. FIG. 9 is another example of a second sub-pattern 14 comprising a continuous area 22 of adhesive in the shape of a cross. In the FIG. 9, the second-sub pattern 14 also comprises an unbonded area 25 at the intersection of the two branches, and a second unbonded area 25 surrounding the continuous area 22 of adhesive.

According to an embodiment, as illustrated in the FIG. 2 to FIG. 6, FIG. 9 and FIG. 10, the first sub-pattern 10 occupies between about 10% and about 40% of the total surface of the laminate 2 or backsheet whereas the second sub-pattern 14 occupies between about 90% and about 60% of the total surface of the laminate 2 or backsheet. In other words, the second sub-pattern 14 represents a substantial portion of the total surface of the laminate 2. The first sub-pattern 10 covers enough surface to ensure the structural integrity of the backsheet and limit delamination and the second sub-pattern covers enough surface to ensure the haptical benefit or soft touch feel of the absorbent article

According to an embodiment, the reinforcement zone 16 occupies up to 5 % of the first sub-pattern 10 area as visible on FIG. 10.

According to an embodiment as illustrated in FIG. 2, the third layer 8 and adhesive pattern comprises a third sub-pattern 26 arranged at least partially in-between the first 10 and second sub-pattern 12, said third sub-pattern 26 comprising a fourth amount of adhesive that is greater than the second amount of adhesive and less than the first amount of adhesive. The third sub-pattern 26 corresponds to a transition zone to help maintain the structural integrity without detriment to the soft touch feel of the backsheet 2. The third sub-pattern 26 is illustrated here as having an elliptical shape that is arranged between the first 10 and the second 14 sub-pattern. The invention is not limited to the shape of the third sub-pattern 26, the transition zone can be rectangular, triangular or in any other polygonal shape, nor to number of third sub-patterns 26, there can be 1, 2, 3, 4, 5, 6, 7, 8, 9, 10 or more third sub-patterns 26.

As explained above, the first amount of adhesive is comprised between about 1 and about 10 gsm. The second amount of adhesive is inferior or equal to about 0.1 gsm. The third amount of adhesive is between about 1.1 gram and about 20 gsm, for example between 10 and 15 gm. The fourth amount of adhesive is between about 0.5 and about 1 gsm. In other words for each square meter of laminate 2 or backsheet 2 there is between about 1 gram and about 10 grams of adhesive in the first sub-pattern 10, 0.1 or less gram of adhesive in the second sub-pattern 14, eventually between 1.1 and 20 grams of adhesive in the reinforcement zones 16 and eventually between about 0.5 and about 1 gram of adhesive in the fourth sub-pattern 26.

As illustrated in FIG. 2, the laminate 2, and thus the backsheet 2, extends longitudinally in a MD direction (Machine direction) and laterally in a CD direction (Cross Direction) and more specifically, the laminate 2 is elongated in the MD direction. The backsheet 2 has thus a front end 28, a rear end 30 and two lateral sides 32. The front end 28 and the rear end 30 are arranged at each longitudinal end in the MD direction and the two lateral sides 32 are arranged at each lateral end in the CD direction. As illustrated in FIG. 2, the first sub-pattern 10, more specifically the frame 12, is divided in at least four panels 12a, 12b, 12c, 12d each having a polygonal shape, here rectangular, with a front panel 12a, a rear panel 12b and two side panels 12c, 12d in regard to the MD direction. The front 12a and rear 12b panels are more extended, or are elongated, in the lateral direction CD than in the longitudinal direction MD and the side panels 12c,12d are more extended, or are elongated, in the longitudinal direction MD than in the lateral directions CD. As illustrated in FIG. 2, the frame 12 and first sub-pattern 10 extend along the entirety of the periphery of the backsheet 2, or in other words, the frame 12 follows the outer contour, or outer edges, of the backsheet 2. It can also be said that the frame 12 is arranged at the front, rear and side edges of the backsheet 2.

The perimeter, periphery, outer contour, rim or outer edges are all elements indicative of the laminate 2, or backsheet 2, once it has been cut and fitted to be a component of the absorbent article, i.e. the final product, as illustrated in FIG. 10 which shows the cut 64 between two laminate 2 units.

FIG. 5 illustrates another embodiment of the arrangement of the first sub-pattern 10, specifically with a frame 12 having a different shape. In this embodiment, the frame 12 comprise four panels 12a, 12b, 12c, 12d arranged at the periphery or outer edges of the backsheet 2, the panels are joined to one another with a bevel 34 arranged on, or at, the inner contour of the frame 12. In other words, the inner surface, or central void, defined by the frame 12 and first sub-pattern 10, has an octagonal shape. As illustrated in FIG. 5, the octagonal shape and thus the area for the second sub-pattern defined by the frame 12 is elongated in the MD direction. Having a bevel 34, or a chamfer, enables to reinforce the corners and the first sub pattern 10 can act as a transition zone at the corners between the reinforcement zone 16 which has a higher amount of adhesive and the second sub-pattern 14 arranged within the inner surface of the frame 12.

As illustrated in FIG. 2, the at least one reinforcement zone 16 is arranged at, or on, or in, the rear end 30 of the backsheet, preferably one at, or on, or in, each lateral side 32. In other words, the two reinforcement zones 16 are arranged on, or in, or at, the rear panel 12d of the frame 12 at each side edges near the corner of the backsheet 2.

FIG. 6 illustrates another embodiment of the laminate 2 from a top view according to the invention, with the second layer 6 on which is applied the third layer adhesive 8 and the first layer 4 (partially illustrated for clarity purposes) superposed on the third layer 8. The second sub-pattern 14 is in the shape of a discontinuous cross, where the discrete areas 20 do not intersect at the centre resulting in a unbonded area 25 in the centre.

As explained above, the invention also pertains to a process for manufacturing a laminate as described above comprising the step of:
- providing a second layer 6 comprising a film;
- applying a third layer 8 of adhesive onto said second layer 6 according to the first 10 and second 14 sub-pattern at least;
- depositing, or superposing, the first layer 4 comprising a nonwoven fabric onto the third layer 8 of adhesive.
The process comprises an additional step of applying fastening ears 24 onto the third layer 8 in an overlapping zone in said reinforcement zone 16. According to an alternative, the process can comprise the step of feeding the first layer 4 of nonwoven, apply the adhesive on it and then deposit the second layer 6 on top of the adhesive.

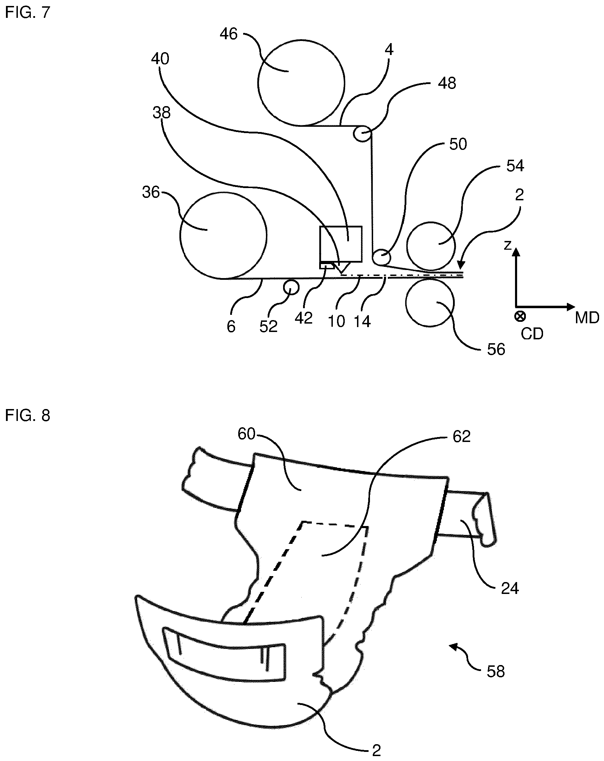

FIG. 7 illustrates schematically from a side-view an apparatus for making a laminated backsheet 2 according to the invention. A roll 36 of thin polymeric material is unwound and the polymer film corresponding to the second layer 6 is conveyed towards a bonding station where the adhesive, or third layer 8, is applied onto the second layer 6. The bonding station comprises at least one nozzle 38, each nozzle 38, for instance, may comprise a slot coating device. The nozzle 38 can receive adhesive from an adhesive reservoir 40.

In one embodiment, the bonding station comprises at least two nozzles 38 which can include a single adhesive orifice or slot. For example, a first nozzle can apply the adhesive in the first sub-pattern 10, a second nozzle can apply the adhesive in the second sub-pattern 14, eventually a third nozzle for the transition zone 26 and eventually a fourth nozzle for the reinforcement zone 16. The orifice or slot may be configured to apply adhesive to a particular location on the film layer 6 or can be configured to apply adhesive over the entire width of second layer 6.

Alternatively, the bonding station can comprise one single nozzle 38 that includes a plurality of spaced apart exit ports or slots for depositing a plurality of continuous and/or discrete areas of adhesive onto the moving web 6. In this embodiment, the single nozzle 38 is configured to apply adhesive in the first 10 and second sub-pattern 14 and eventually third sub-pattern 26 and reinforcement zones 16.

The bonding station further comprises an adhesive flow control device 42 to control the flow of adhesive from the reservoir 40 to the one or more exit ports on the nozzle. The flow control device 42 can comprise, for instance, any suitable valve or similar device to control the flow of adhesive. In one embodiment, for instance, the adhesive flow control device 42 comprises an electromagnetic valve, such as a solenoid valve. In one embodiment, the adhesive flow control device 42 can be in communication with a controller, such as one or more microprocessors. The controller can be configured to control the adhesive flow control device 42 for enabling or disabling the flow of adhesive through the nozzle at desired times, such as during periodic intervals. For example, the adhesive flow control device 42 may open the valve to allow the adhesive to flow through the nozzle 38 and may close the valve to cease the flow of adhesive through the nozzle.

As explained above, the bonding station can include a single nozzle 38 or can include multiple nozzles 38. Additionally, the bonding station can include a single adhesive flow control device 42 or multiple adhesive flow control device 42. For instance, if the nozzle 38 includes a plurality of exit ports, the bonding station can include a corresponding plurality of adhesive flow control device 42, or valves, for controlling each of the individual exit ports. Alternatively, a single adhesive flow control device 42 can control flow through all of the exit ports simultaneously.

The adhesive, or third layer 8, is applied on the second layer 6 according to the first 10 and second 14 sub-patterns at least as described above and eventually the third sub-pattern 26 and reinforcement zones 16.

A roll 46 of nonwoven fabric is unwound and the nonwoven web corresponding to the first layer 4 is conveyed towards the second and third layer 8. The apparatus of the present disclosure can include web conveying devices for conveying webs of material, such as the film web, in other words the second layer 6, and the nonwoven web, in other words the first layer 4. As shown in FIG. 7, the web conveying device can include one or more guide rollers 48, 50, 52 for supporting and convey the webs of film 6 and/or nonwoven material 4.

The first layer 4 is superposed onto the third 8 and second layer 6 to form the backsheet 2. The apparatus can optionally comprise a lamination station as illustrated in FIG. 7, where the superposed first 4, third 8 and second 6 layers pass between two rollers 54,56 forming a nip where the backsheet 2 passes thereby pressing, and further laminating, the layers 4,6,8 altogether. Alternatively, the guiding roller 50 illustrated in FIG. 7 can be arranged in a way to guide the first layer 4 up against the third layer 8.

To summarize, the process for manufacturing a laminate as described above comprises the step of:
- providing the second layer 6 comprising a polymeric film;
- applying the third layer 8 of adhesive onto said second layer 6 according to the first and second sub-pattern 10,14 at least preferably with, or by, an electromagnetic actuator;
- depositing the first layer 4 comprsing a nonwoven fabric onto the third layer 8 of adhesive.

Alternatively, the process for manufacturing a laminate as described above comprises the step of:
- providing the first layer 4 comprising a nonwoven fabric;
- applying the third layer 8 of adhesive onto said first layer 4 according to the first and second sub-pattern 10,14 at least preferably with, or by, an electromagnetic actuator;
- depositing said second layer 6 comprising a film onto the third layer 8 of adhesive.

The rest of the process and apparatus, not illustrated, comprise additional steps such as the addition of a topsheet, the addition of an absorbent core in between, the deposit of ears 24, or elastic tabs or fastening tabs, onto the backsheet 2 at the reinforcement zones 16 and a cutting step where the absorbent articles are separated from one another. These steps can be in this order or in another order.

According to one embodiment, the fastening tabs 24 can be deposited onto the reinforcement zones 16 before the first layer 4 is superposed on the third layer 8. According to another embodiment, the first layer 4 can comprise a cut out the dimension of the reinforcement zone so that the elastic tabs can be arranged onto the reinforcement zones. According to another embodiment, the second layer 6 can have an extension that can be covered by the third layer but not by the first layer. According to another embodiment, an additional layer of adhesive is added on the first layer 4 so as to fix the ear 24 onto the first layer 4.

As illustrated in FIG. 8, the invention also pertains to an absorbent article 58 comprising a topsheet 60, a backsheet 2 comprising the laminate 2 as described above and an absorbent core 62 arranged in between topsheet 60 and backsheet 2. The topsheet 60 is arranged to be the body-facing surface and is intended to be disposed toward or placed adjacent to the body of the wearer during ordinary use. The backsheet 2 and laminate 2 are arranged to be the garment-side surface and is intended to be disposed to face away from the wearer's body during ordinary use and placed adjacent to the wearer's undergarments when the absorbent article 58 is worn.

The backsheet 2, in particular the film of the second layer 6, may comprise material that is breathable so as to allow vapour to escape from the absorbent material, while still preventing liquids from passing there through.

The absorbent article 58 may further comprise additional components that are common in the art and selected from the group consisting of: a liquid distribution layer (ADL) positioned between the topsheet 60 and the absorbent core 62; elastic or non-elastic back ears 24 joined to the chassis at a position proximal to the rear end 30 of the laminate 2 forming the backsheet; super absorbent polymer particles and/or fibers typically comprised within the absorbent core 62; cellulose fibers typically comprised within the absorbent core 62; fastening tapes joined to the back ears for adhesive coupling with a garment facing surface of the backsheet at a position proximal to the first end; elastic waistband positioned proximal to the first and/or second ends; containment gaskets; barrier cuffs arranged along the lateral sides of the absorbent article and combinations thereof. When the absorbent article is a pant, the absorbent article may comprise a front belt positioned proximal to the first end and a rear belt positioned proximal to the second end, said belts being separated from each other by the chassis of the absorbent article and being directly joined to each other via two opposite side seams, said belts typically being elastic.

The amount of adhesive in the first sub pattern 10, second sub-pattern 14, third sub-pattern 26 and reinforcement zones 16 can be measured on a gram per square meter (gsm) basis. When measuring the amount of adhesive in the different sub-patterns based on a gram per square meter basis, the total weight of adhesive in grams in a particular sub-pattern can be divided by the surface or area (length x width) of said particular sub-pattern in the machine direction and in the cross direction.

For instance, the amount of adhesive in the first sub-pattern 10 can be measured by taking a sample of the web (film or NW fabric) where the first sub-pattern 10 is arranged, weighing said sample before and after removal of the adhesive (in grams) the difference giving the weight of the adhesive, and dividing the weight of adhesive by the area of the sample (in m²) thereby obtaining the amount of adhesive in gsm. For example, the adhesive can be removed by using a solvent such as hexane. A similar analysis can be conducted in order to determine the amount of adhesive present in the second sub-pattern 14, third sub-pattern 26 or reinforcement zones 16 in grams per square meter.

It is supposed that the present invention is not restricted to any form of realization described previously and that some modifications can be added to the presented example of fabrication without reappraisal of the appended claims.

## Claims

1. Laminate (2) for an absorbent article, preferably consisting of a backsheet of said absorbent article, said laminate (2) comprising:
- a first layer (4) comprising a fibrous material such as a nonwoven fabric;
- a second layer (6) comprising a film;
- a third layer (8) comprising an adhesive;
- the third layer (8) being arranged between the first (4) and second (6) layer;
wherein the adhesive in the third layer (8) is applied in a pattern, said pattern comprising:
- a first sub-pattern (10) comprising a frame (12) following the perimeter of the laminate (2) and defining an inner surface, said first sub-pattern (10) comprising a first amount of adhesive;
- a second sub-pattern (14) arranged within the inner surface of the frame (12), said second sub-pattern (14) comprising a second amount of adhesive, the second amount of adhesive being less than the first amount of adhesive.

2. Laminate (2) according to claim 1, wherein the first amount of adhesive is at least five times greater than the second amount of adhesive.

3. Laminate (2) according to any of the preceding claims, wherein the first amount of adhesive is comprised between 0.5 and 5 gsm.

4. Laminate (2) according to any of the preceding claims, wherein the second amount of adhesive is under 0.1 gsm.

5. Laminate (2) according to any of the preceding claims, wherein the first sub-pattern (10) comprising the frame (12) comprises at least one additional reinforcement zone (16), comprising a third amount of adhesive that is greater than the first amount of adhesive.

6. Laminate (2) according to claim 5, wherein the third amount of adhesive is at least two times greater than the first amount of adhesive.

7. Laminate (2) according to any of the preceding claims, wherein the second sub-pattern (14) comprises discrete areas (20) of adhesive, the discrete areas (20) of adhesive being arranged in a staggered configuration or in an even distribution.

8. Laminate (2) according to any of the preceding claims, wherein the second sub-pattern (14) comprises discrete areas (20) of adhesive, the discrete areas (20) of adhesive being in shape of dots or rectangles.

9. Laminate (2) according to any of the preceding claims, wherein the first sub-pattern (10) occupies between 10 and 40 % of the absorbent article total surface whereas the second seb-pattern (14) occupies between 90 and 60 % of the absorbent article total surface.

10. Laminate (2) according to any of the preceding claims, wherein the pattern of the third layer (8) comprises a third sub-pattern (26) arranged at least partially in-between the first (10) and second (14) sub-pattern, said third sub-pattern (26) comprising a fourth amount of adhesive that is greater than the second amount of adhesive and less than the first amount of adhesive.

11. Laminate (2) according to any of the proceeding claims, wherein the laminate (2) extends longitudinally in a MD direction and laterally in a CD direction, said laminate (2) comprising a front end (28) and a rear end (30) arranged at each longitudinal end and two lateral sides (32) arranged at each lateral end, and
wherein the frame (12) is divided in at least four panels (12a,12b,12c,12d) having polygonal shape: a front panel (12a), a rear panel (12b) and two side panels (12c,12d),
i. wherein the front (12a) and rear (12b) panels extend more in the lateral direction (CD) than in the longitudinal direction (MD) and
ii. the side panels (12c,12d) extend more in the longitudinal direction (MD) than in the lateral direction (CD).

12. Laminate (2) according to claim 12, wherein the at least one reinforcement zone (16) is arranged on the rear panel (12b).

13. Laminate (2) according to any of the claim 12 or 13, wherein the panels (12a, 12b, 12c, 12d) are joined to one another with a bevel (34) arranged on the inner contour of the frame (12).

14. Absorbent article (58) comprising:
- a topsheet (60);
- a backsheet comprising a laminate (2) according to any of claims 1 to 13; and
- an absorbent core (62) arranged in between said topsheet (60) and said backsheet.

15. Process for manufacturing a laminate (2) according to any of claims 1 to 13 comprising the step of:
- providing respectively the second layer (6) comprising a film or the first layer (4) comprising a nonwoven fabric ;
- applying the third layer (8) of adhesive respectively onto said second layer (6) or onto said first layer (4) according to the first (10) and second (14) sub-pattern at least preferably with an electromagnetic actuator;
- depositing respectively said first layer (4) or said second layer (6) onto the third layer (8) of adhesive.
